# EUROPÄISCHE PATENTSCHRIFT

(11) **EP 1 518 851 B1**
(45) Veröffentlichungstag und Bekanntmachung des Hinweises auf die Patenterteilung: **22.11.2006**
(21) Anmeldenummer: 04022005.5
(22) Anmeldetag: 16.09.2004
(51) Int. Cl.: C07C 41/01

(54) **Verfahren zur Herstellung flüssigkristalliner Ether**
Process for the preparation of liquid crystalline ethers
Procédé pour la préparation d'éthers liquides cristallins

(30) Priorität: 25.09.2003 EP 03021634
(43) Veröffentlichungstag der Anmeldung: 30.03.2005
(73) Patentinhaber: AZ Electronic Materials (Germany) GmbH, 65203 Wiesbaden (DE)
(72) Erfinder: Wingen, Rainer, Dr., 65719 Hofheim (DE)
(74) Vertreter: Isenbruck, Günter

(56) Entgegenhaltungen:
- EP-A- 0 030 761
- EP-A- 1 061 113
- DATABASE WPI Section Ch, Week 197544 Derwent Publications Ltd., London, GB; Class E17, AN 1975-73448W XP002270420 & JP 50 031126 B (OSAKA PREFECTURE) 7. Oktober 1975 (1975-10-07)
- S.W. BALDWIN ET AL.: "Reductive deoxygenation of esters with trichlorosilane." JOURNAL OF ORGANIC CHEMISTRY., Bd. 40, Nr. 26, 1975, Seiten 3885-3887, XP002270417 EASTON US
- KUNIO OKA ET AL.: "Gamma ray induced reduction of sterically hindered alkyl carboxylates with trichlorosilane in the presence of hydrogen chloride" JOURNAL OF ORGANOMETALLIC CHEMISTRY., Bd. 362, 1989, Seiten 31-35, XP002270418 ELSEVIER-SEQUOIA S.A. LAUSANNE., CH ISSN: 0022-328X
- ZHIBIAO MAO ET AL.: "Catalytic hydrosilation of organic esters using manganese carbonyl acetyl complexes, (L)(CO)4MnC(O)CH3 (L = CO, PPh3)" JOURNAL OF THE AMERICAN CHEMICAL SOCIETY., Bd. 117, Nr. 40, 1995, Seiten 10139-10140, XP002270419 DC US
- NAKAO R ET AL: "REDUCTION WITH TRICHLOROSILANE. III CYCLIC ETHER FROM LACTONE" JOURNAL OF ORGANIC CHEMISTRY, AMERICAN CHEMICAL SOCIETY. EASTON, US, Bd. 37, Nr. 1, 14. Januar 1972 (1972-01-14), Seiten 76-78, XP000569711 ISSN: 0022-3263
- TSURUGI, JITSUO ET AL.: "A novel gamma induced reduction with trichlorosilane. Dialkyl ether from alkyl aliphatic carboxylate" JOURNAL OF THE AMERICAN CHEMICAL SOCIETY., Bd. 91, Nr. 16, 1969, Seiten 4587-4588, XP002299574 USAMERICAN CHEMICAL SOCIETY, WASHINGTON, DC.

## Beschreibung

Konstituenten von Flüssigkristallmischungen, bei denen Cyclohexyl- bzw. Bicyclohexyl-Einheiten über eine Ether-Gruppe - d.h. die Gruppierung -CH₂O- - miteinander verbunden sind, sind z.B. aus US 4,361,494 und EP-A-1 061 113 bekannt.
Auch Verfahren zur Herstellung dieser Verbindungen werden dort genannt, insbesondere die Reduktion von Carbonsäureestern mit Borverbindungen ( EP-A-1 061 113, S. 6, Schema 1 und S.27, Beispiel 1, Schritt 1.2; US 4,361,494, p. 6, example 2) sowie die Ethersynthese nach Williamson (US 4,361,494, p.8, (C) ).

Die Literatur (z.B. R.C.Larock, Comprehensive Organic Transformations: A Guide to Functional Group Preparations, VCH Publ., N.Y., 1989, ISBN 0-89573-710-8, transformation index pp.1110-1111; J. March, Advanced Organic Chemistry, McGraw-Hill, ed., 1977, ISBN 0-07-040247-7, classification of reactions by type of compound synthesized p.1179) kennt eine Vielzahl von Verfahren, um Verbindungen mit einer Ethergruppe -CH₂O-herzustellen.
Da zur Erzielung der flüssigkristallinen Eigenschaften bei Cyclohexanderivaten die *trans-*Anordnung erforderlich ist, scheiden Verfahren aus, bei denen nicht ein hoher Anteil an *trans*-Verbindungen erzielt wird oder - bei Einsatz von *trans*-Derivaten als Ausgangsmaterialien- beibehalten werden kann. Insofern ist davon auszugehen, dass die in EP-A-1 061 113 bzw. US 4,361,494 genannten Syntheseverfahren bereits eine Optimierung hinsichtlich dieser bei Flüssigkristallen mit Cyclohexan-Baugruppen gegebenen Notwendigkeit darstellen (z.B. eine dementsprechende engere Auswahl: R.C.Larock, Comprehensive Organic Transformations: A Guide to Functional Group Preparations, VCH Publ., N.Y., 1989, ISBN 0-89573-710-8, pp. 446-448, 471; J. March, Advanced Organic Chemistry, McGraw-Hill, ed.,1977, ISBN 0-07-040247-7; pp. 357-358, 1122).

Wie sich bei der Durchführung der Reduktion mit Borverbindungen gemäß EP-A-1 061 113 zeigt, kann der "normale" Verlauf der Reduktion eines Esters unter Bildung zweier Alkohole, wie er durch Schema 1 verdeutlicht wird, nicht vollständig unterdrückt werden. Durch diese Konkurrenzreaktion unter Bildung der Alkohole (X) und (XI) bleibt die Ausbeute von (I) gering. Das Verfahren ist nicht ökonomisch wegen dieser geringen Ausbeute, dem folglich hohen Verbrauch teuren all-trans-Ausgangsmaterials (II) sowie der geringen Raum-Zeit-Ausbeute (hohe Verdünnung und schlechte Ausbeute).
Bei der Ethersynthese nach Williamson (US 4,361,494) gemäß Schema 2 wird ebenfalls eine geringe Ausbeute an (I) erhalten. Zusätzlich müssen gemäß US 4,361,494 die Verbindungen des Typs (XX) erst hergestellt werden, indem die entsprechenden 4-Alkylcyclohexancarbonsäuren bzw. analog die 4'-Alkyl-1,1'-bicyclohexan-4-carbonsäuren durch Reduktion mit Metallhydriden zu den jeweiligen Hydroxymethylverbindungen umgesetzt werden [US 4,361,494, p. 7, (A)], die anschließend in einer weiteren Reaktionsstufe [US 4,361,494, p. 8, (C)] die entsprechenden Brommethylverbindungen (XX) ergeben. Ausbeuteverluste über drei Synthesestufen sowie drei Synthesestufen an sich (mit der Notwendigkeit der Isolation von Zwischenstufen, zusätzlicher Reinigungsoperationen etc.) machen dieses Verfahren ebenfalls unökonomisch.

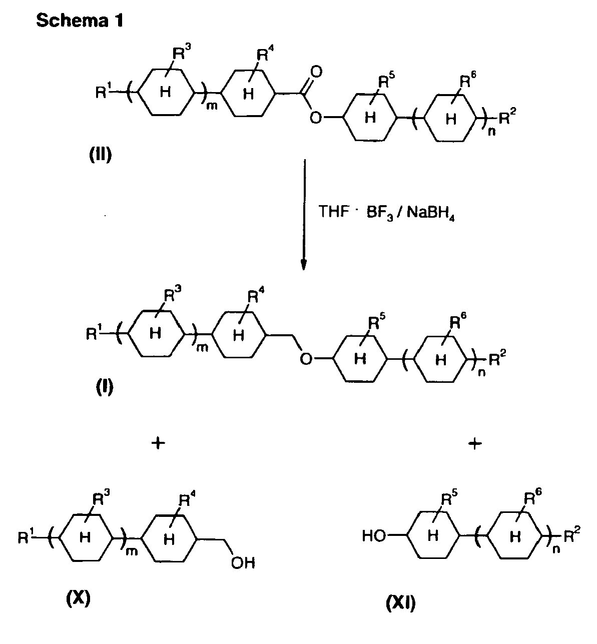

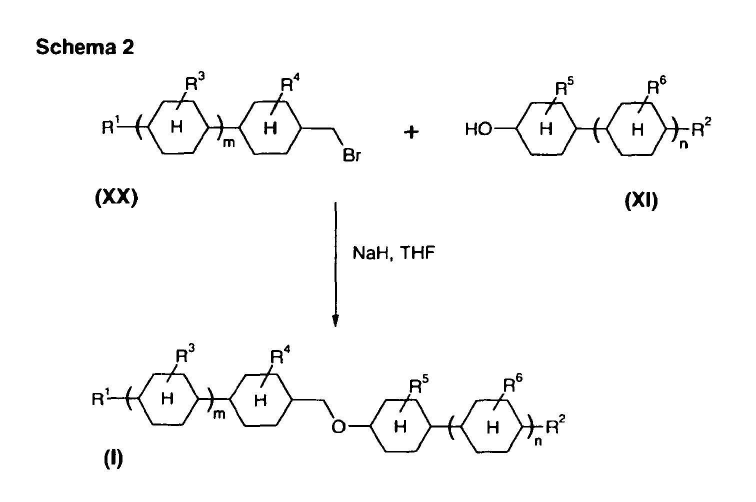

Es besteht daher ein Bedarf an einem ökonomischen Herstellverfahren, das durch höhere Ausnutzung der flüssigkristallinen Ausgangsmaterialien (II), höhere Ausbeute an (I), höhere Raum-Zeit-Ausbeute sowie kostengünstige Reaktanden gekennzeichnet ist.

Da die Anzahl der Synthesestufen ausgehend von der 4-Alkyl-cyclohexancarbonsäure bzw. 4'-Alkyl-1,1'-bicyclohexan-4-carbonsäure im Falle der Reduktionsverfahren geringer ist als im Falle der Ethersynthese nach Williamson, stand zur Lösung dieser Aufgabe die Suche nach anderen Reduktionsbedingungen bzw. Reduktionsmitteln im Vordergrund.
Einige der in der Literatur beschriebenen Verfahren sind jedoch nur für cyclische Ester geeignet, z.B. die Reduktion mit
a) H₂, PtO₂ : Chem. Ind. 975 (1964)
b) i-Bu₂AlH / Et₃SiH, BF₃ · OEt₂ : J. Org. Chem 46, 2417 (1981)
c) Cl₃SiH: J.Org. Chem. 37, 76 (1972); J.Org. Chem. 39, 2470 (1974)
d) LiAlH₄, AlCl₃ : Chem. Ind. 230 (1977),
   oder erfordern teure und nicht technisch verfügbare Einsatzstoffe, z.B.
e) PhSiH₃, (PPh₃)(CO)₄MnC(=O)CH₃ : J. Am. Chem. Soc. 117, 10139 (1995).

Bei der Reduktion der Ester cyclischer Alkohole (1-Adamantyl- acetat und -pivalat; Cyclododecan-acetat) mittels Cl₃SiH gemäß J.Org.Chem 40, 3885 (1975) werden geringe Ausbeuten der entsprechenden Ether (2-42% 1-Adamantan-ethylether, 1-12% 1-Adamantanneopentylether; 67% Cyclododecyl-ethylether; Ausbeuten jeweils nach GC) erhalten, wohingegen die analoge Reduktion der Ester linerarer Alkohole (Dodecylacetat) Ausbeuten von 98% verzeichnet. Hieraus lässt sich schließen, dass sich Ester, bei denen die Alkohol- oder Säure-Komponente einen Cyclus enthält, schlecht zu den entsprechenden Ethern reduzieren lassen.

Es wurde nun erfindungsgemäß gefunden, dass die Reduktion von Estern der Formel (II) - bei denen Alkohol- und Säure-Komponente einen Cylohexanrest enthalten - mit Silanen (III) unter radikalischen Bedingungen zu hohen Ausbeuten an Ethern (I) führt, wobei die Reaktion 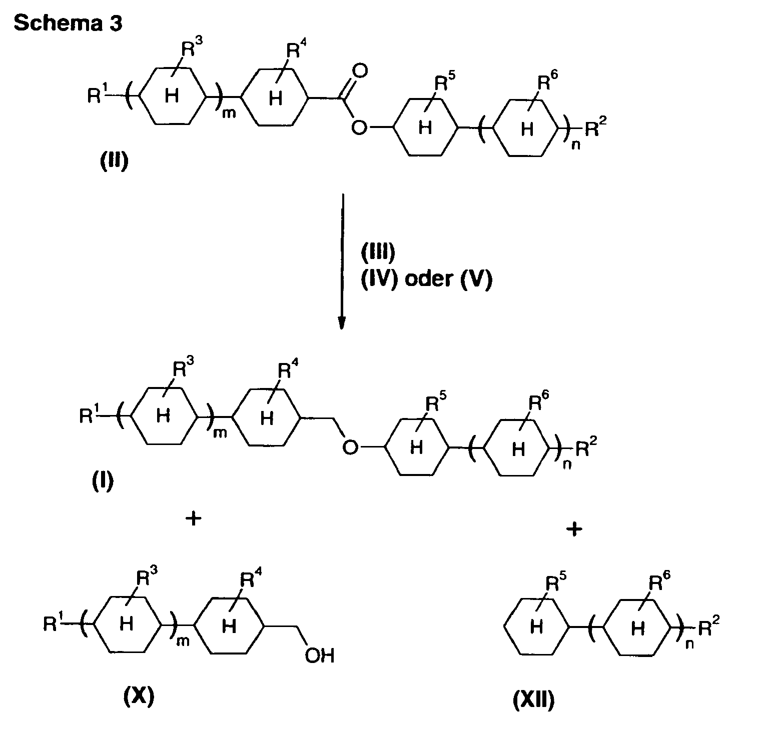 durch hohe Konzentrationen und kurze Reaktionszeiten und folglich durch eine sehr hohe Raum-Zeit-Ausbeute gekennzeichnet ist.

Die Erfindung betrifft somit ein Verfahren zur Herstellung von Ether-Verbindungen der Formel (I) durch Reduktion von Carbonsäure-Estern der Formel (II), bei dem 1 Mol-Äquivalent einer Verbindung der Formel (II) mit 2 bis 6 Mol-Äquivalenten eines Silans der Formel (III),

**(III) :** R¹¹R¹²R¹³SiH

in einer Radikalkettenreaktion, gegebenenfalls in einem Lösemittel, umgesetzt wird, wobei in den Formeln die Reste bedeuten:
**R**^{**1**}**, R**^{**2**} **:** jeweils unabhängig voneinander Wasserstoff oder einen unsubstituierten, einen einfach durch CN oder CF₃ oder mindestens einfach durch Halogen substituierten Alkyl-Rest mit 1 bis 12 C-Atomen oder Alkenyl-Rest mit 2 bis 12 C-Atomen, wobei in diesen Resten auch eine oder mehrere -CH₂-Gruppen jeweils unabhängig voneinander durch -O-, -S-, Cyclopropan-1,2-diyl, Cyclobutan-1,3-diyl so ersetzt sein können, dass O-Atome nicht direkt miteinander verknüpft sind,
mit der Maßgabe, dass von R¹, R² nur eines Wasserstoff sein darf
**R**^{**3**}**, R**^{**4**}**, R**^{**5**}**, R**^{**6**} jeweils unabhängig voneinander Wasserstoff, Halogen, Methyl oder Cyano **R**^{**11**}**, R**^{**12**}**, R**^{**13**} jeweils unabhängig voneinander Wasserstoff, Chlor, Alkyl mit 1 bis 8 C-Atomen, Alkyloxy mit 1 bis 8 C-Atomen, Cyclohexyl, Methylcyclohexyl, Cyclopentyl, Phenyl, oder R¹¹ und R¹² zusammen die Gruppierung -(CH₂)ₚ- mit der Maßgabe, dass mindestens eines von R¹¹, R¹², R¹³ Chlor bedeutet,
**m, n** jeweils unabhängig voneinander Null oder 1,
**p** 4 oder 5.

Dabei liegt das Verhältnis der mol-Äquivalente von (II) : (III) vorzugsweise im Bereich von 1:2 bis 1:5.

Die Radikalkettenreaktion wird vorzugsweise durch freie Radikale ausgelöst, die bei der Umsetzung durch Zerfall von Radikalstartern, durch aktinische Strahlung oder eine Kombination davon gebildet werden. Es können beispielsweise Radikalstarter eingesetzt werden, die thermisch und/oder photochemisch unter Bildung freier Radikale zerfallen.

Vorzugsweise werden Radikalstarter der Formel (IV) bzw. (V) eingesetzt

**(IV):** R²¹OOR²²

**(V):** R³¹-N=N-R³²

mit der Bedeutung
**R**^{**21**} **, R**^{**22**} Wasserstoff oder ein (gegebenenfalls durch Alkylperoxy- oder Hydroperoxy-Gruppen substituierter) Alkyl- oder Alkandiyl-Rest mit 1 bis 8 C-Atomen oder ein (gegebenenfalls durch Alkyl- und/oder Alkylperoxy- und/oder Hydroxy- und/oder Hydroperoxy-Gruppen substituierter) Cyclohexanring, mit der Maßgabe, dass nur eines von R²¹, R ²² Wasserstoff bedeuten kann
**R**^{**31**} **R**^{**32**} jeweils unabhängig voneinander R⁴¹R⁴²C(X), worin R⁴¹, R⁴² unabhängig voneinander eine Alkyl- oder Alkyloxygruppe mit 1-8 C-Atomen und X -CN oder -C(=O)OR⁴³ bedeutet, mit R⁴³=Alkylgruppe mit 1 bis 8 C-Atomen.

In den Formeln (I) und (II) bedeuten R¹ und R² jeweils unabhängig voneinander vorzugsweise C₂₋₇-Alkyl, insbesondere C₃₋₅-Alkyl. Die Alkylreste können dabei verzweigt oder vorzugsweise linear sein.

Die Reste R³, R⁴, R⁵ und R⁶ bedeuten vorzugsweise unabhängig voneinander Wasserstoff oder Halogen.

Vorzugsweise ist die Summe von n und m 0 oder 1, d.h. entweder hat m den Wert 0 und n den Wert 1, oder m hat den Wert 1 und n hat den Wert Null.

Das erfindungsgemäße Verfahren gemäß Schema 3 setzt die leicht zugänglichen (vgl. EP-A-1 061 113) Ester der Formel (II) ein, die in einer Radikalkettenreaktion mit Silanen der Formel (III) umgesetzt werden. R¹¹ und R¹² sind dabei vorzugsweise Chlor. Die Anwesenheit von Radikalen kann durch Radikalstarter oder aktinische (z.B. γ-) Strahlung bewirkt werden. Werden Radikalstarter, z.B. der Formeln (IV) oder (V), eingesetzt, kann die Initiierung der Radikalkettenreaktion (durch Zerfall der Radikalstarter) mittels Zuführung thermischer oder photochemischer Energie erfolgen. Als Nebenreaktion kann die sogenannte "reduktive Deoxygenierung" beobachtet werden, die zu Verbindungen des Typs (X) und (XII) führt.

Die Einstrahlung photochemischer Energie kann mittels Tauchlampen innerhalb des Reaktionsgefässes oder durch eine für die gewählte Wellenlänge transparente Öffnung im Reaktionsgefäß von außen erfolgen. Bevorzugt werden Tauchlampen eingesetzt.

Als Silane (III) können vorzugsweise eingesetzt werden: Trichlorsilan, Dichlor-alkyl-silane, Cyclohexyldichlorsilan, Chlor-dialkylsilane, Chlor-alkyl-cyclohexylsilane, Dichlor-phenylsilan, Diphenyl-chlor-silan, Alkoxy-dichlorsilane, Dialkoxy-chlor-silane. Bevorzugt sind Trichlorsilan, Dichlor-alkylsilane, Cyclohexyldichlorsilan und Dichlor-phenylsilan, wobei unter den Alkylresten C₁₋₆-Alkyl, insbesondere Methyl und Ethyl bevorzugt werden. Ganz besonders bevorzugt wird Trichlorsilan.

Als Radikalstarter (IV) können großtechnisch verfügbare (z.B. Handelsnamen Trigonox oder Luperox) Hydroperoxide und Peroxide eingesetzt werden, die im Reaktionsmedium löslich sind und selbst bzw. in Form ihrer durch radikalischen Zerfall entstehenden Spaltprodukte gegenüber (II), (III) und (I) chemisch nur in untergeordnetem-Maße aktiv sind. Beispiele sind: tert-Butylhydroperoxid; 1-Hydroperoxy-1'-hydroxydicyclohexylperoxid; Di-tertbutylperoxid; Di-tert-amylperoxid; 2,2-Di-(tert-butylperoxy)butan; 1,1-Bis-(tert-amylperoxy)cyclohexan; 1,1-Di-(tert-butylperoxy)cyclohexan; 1,1-Bis(tert-butyl)-3,3,5-trimethylcyclohexanperoxid; 3,6,9-Triethyl-3,6,9-trimethyl-1,4,7-triperoxynonan.

Als Radikalstarter (V) können z.B. eingesetzt werden: Azo-bis-isobutyronitril, Dimethyl-2,2'-azobisisobutyrat (Handelsname V-601).

Lösungsmittel, die sich zur Durchführung der Reaktion eignen, müssen gegenüber den Reaktionspartnern inert sein und über ein gutes Lösevermögen für die Edukte verfügen. In diesem Sinne geeignete Lösungsmittel sind Ether wie Tetrahydrofuran, 2-Methyltetrahydrofuran, 1,4-Dioxan, Ethylenglykoldialkylether, tert-Butylmethylether oder Kohlenwasserstoffe wie Cyclohexan, Methylcyclohexan, Hexan, Heptan, Isooctan oder Chlorkohlenwasserstoffe wie Dichlormethan. Bevorzugt wird Tetrahydrofuran.

Die Reaktion kann in weiten Temperaturbereichen durchgeführt werden, gegebenenfalls unter Anwendung von Druck, falls die Reaktionstemperatur oberhalb des Siedepunktes der niedrigst siedenden Komponente liegt. Bevorzugt wird eine Reaktionstemperatur zwischen -5 und 30°C, besonders bevorzugt zwischen 10 und 25°C, im Falle der photochemischen Initiierung der Radikalbildung. Im Falle der thermischen Initiierung der Radikalbildung ist die Reaktionstemperatur (bzw. der Druck) so zu wählen, dass der Zerfall des Radikalstarters in deutlichem Umfang bei einer Temperatur unterhalb des Siedepunktes des Lösemittels erfolgt.

Die Reaktion kann in weiten Konzentrationsbereichen durchgeführt werden; bevorzugt wird mit der Sättigungskonzentration von (I) in dem jeweiligen Lösungsmittel bei einer Temperatur von bis zu 10K unterhalb der Reaktionstemperatur gearbeitet.

Neben der deutlich höheren Ausbeute an (I) hat das erfindungsgemäße Verfahren zusätzlich den Vorteil, dass Silane, insbesondere Trichlorsilan, kostengünstige großtechnische Produkte sind. Ferner können infolge kurzer Reaktionszeiten und hoher Konzentrationen (gute Löslichkeit von Edukt und Produkt z.B. in THF) hohe Raum-Zeit-Ausbeuten erzielt werden. Die Summe aller drei Gegebenheiten macht das erfindungsgemäße Verfahren für eine technische Umsetzung besonders geeignet.

Die Erfindung betrifft auch ein Verfahren zur Herstellung von Ether-Verbindungen der Formel (XXI) 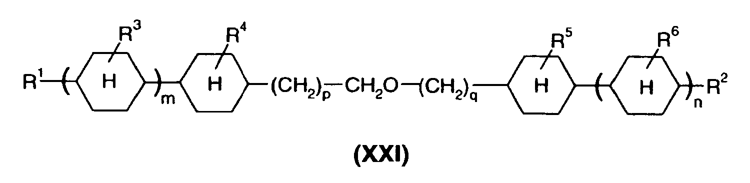 durch Reduktion von Carbonsäure-Estern der Formel (XXII), 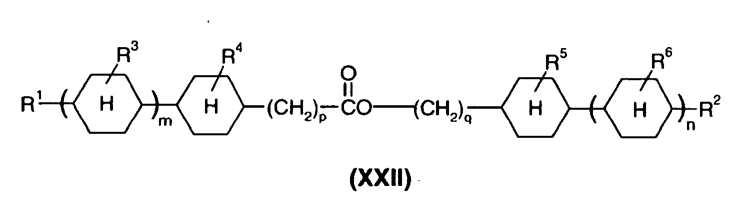 wobei 1 Mol-Äquivalent einer Verbindung der Formel (XXII) mit 2 bis 6 Mol-Äquivalenten eines Silans der Formel (III)

**(III)** R¹¹R¹²R¹³SiH

in einer Radikalkettenreaktion, gegebenenfalls in einem Lösemittel, umgesetzt wird; wobei in den Formeln die Reste bedeuten:
**R**^{**1**}**, R**^{**2**} **:** jeweils unabhängig voneinander Wasserstoff oder einen unsubstituierten oder einfach durch CN oder CF₃ oder mindestens einfach durch Halogen substituierten Alkyl-Rest mit 1 bis 12 C-Atomen oder Alkenyl-Rest mit 2 bis 12 C-Atomen, wobei in diesen Resten auch eine oder mehrere -CH₂-Gruppen jeweils unabhängig voneinander durch -O-, -S-,
Cyclopropan-1,2-diyl, Cyclobutan-1,3-diyl so ersetzt sein können, dass O-Atome nicht direkt miteinander verknüpft sind,
mit der Maßgabe, dass von R¹, R² nur eines Wasserstoff sein darf
**R**^{**3**}**, R**^{**4**} **, R**^{**5**}**, R**^{**6**} jeweils unabhängig voneinander Wasserstoff, Halogen, Methyl oder Cyano
**R**^{**11**}**, R**^{**12**}**, R**^{**13**} jeweils unabhängig voneinander Wasserstoff, Chlor, Alkyl mit 1 bis 8 C-Atomen, Alkyloxy mit 1 bis 8 C-Atomen, Cyclohexyl, Methylcyclohexyl, Cyclopentyl, Phenyl,
mit der Maßgabe, dass mindestens eines von R¹¹, R¹², R¹³ Chlor bedeutet,
**m, n** jeweils unabhängig voneinander Null oder 1,
**p und q** jeweils unabhängig voneinander 0, 1, 2 oder 3, die Summe p+q 1, 2 oder 3.

Bevorzugte Bedeutungen der Reste sowie bevorzugte Reaktionsbedingungen sind wie vorstehend für (I) angegeben.

Das Verfahren wird durch die nachstehenden Beispiele näher erläutert, ohne darauf eingeschränkt zu sein.
Um die verschiedenen Verfahren mit ihren unterschiedlichen Verunreinigungsprofilen - und folglich unterschiedlichen, auch in ihrer Effizienz unterschiedlichen Isolationstechniken und Reinigungsverfahren - besser vergleichen zu können, wurde die Bewertung der Verfahren anhand der Kennzahlen Rohmasse und Gehalt per GC (für alle Verfahren einheitlich), bestimmt an der Rohware nach Entfernung der Reaktionspartner und der Hilfsstoffe, vorgenommen.

### Beispiele

### Beispiel 1

### Herstellung von all-trans-4-[[(4-Propylcyclohexyl)methyl]oxy]-4'-pentyl-1,1'-bicyclohexan [Verbindung (I) mit R¹ = Propyl, R²= Pentyl, R³ = R⁴ = R⁵ = R⁶ = H, m=0, n = 1]

Eine Lösung von 26,26g (65 mmol) *all-trans-* 4-Propylcyclohexancarbonsäure-4-(4'-pentyl-1,1'- bicyclohexan]ester [(II) mit R¹ = Propyl, R²= Pentyl, R³ = R⁴ = R⁵ = R⁶ = H, m=0, n = 1] in 130 ml Tetrahydrofuran wird mit 35,3g (260 mmol) Trichlorsilan (III) sowie 4,8g (32,4 mmol) Di-tertbutylperoxid (IV) versetzt und für 3 h bei 20°C dem Licht einer Mitteldruck-UV-Lampe (Tauchlampe, Quarzrohr, 150W) ausgesetzt. Anschließend wird die Reaktionsmischung auf 500 ml Eiswasser gegossen, 100 ml 32%ige Natriumhydroxidlösung werden zugesetzt, und es wird mit 100 ml tert-Butylmethylether extrahiert. Die wäßrige Phase wird noch zweimal mit je 100 ml tert-Butylmethylether extrahiert.Die organischen Phasen werden vereinigt, und die Lösemittel werden im Vakuum abdestilliert. Das Rohprodukt wird der Analytik per GC unterzogen.

### Beispiel 2

### Herstellung von all-trans-4-[[(4-Propylcyclohexyl)methyl]oxy]-4'-pentyl-1,1'-bicyclohexan [Verbindung (I) mit R¹ = Propyl, R²= Pentyl, R³ = R⁴ = R⁵ = R⁶ = H, m=0, n = 1]

Es wird wie in Beispiel 1 vorgegangen, jedoch mit einem Verhältnis (II) : (III) von 1: 2,5 gegenüber 1: 4 in Beispiel 1.

### Beispiel 3

### Herstellung von all-trans-4-[[(4-Propylcyclohexyl)methyl]oxy]-4'-pentyl-1,1'-bicyclohexan [Verbindung (I) mit R¹ = Propyl, R²= Pentyl, R³ = R⁴ = R⁵ = R⁶ = H, m=0, n = 1]

Es wird wie in Beispiel 1 vorgegangen, jedoch unter Verwendung von Chlor-dimethylsilan mit einem Verhältnis (II) (III) von 1: 3.

### Beispiel 4

### Herstellung von all-trans-4-[[(4-Propylcyclohexyl)methyl]oxy]-4'-pentyl-1,1'-bicyclohexan [Verbindung (I) mit R¹ = Propyl, R²= Pentyl, R³ = R⁴ = R⁵ = R⁶ = H, m=0, n = 1]

Es wird wie in Beispiel 1 vorgegangen, jedoch unter Verwendung von Dichlor-ethylsilan mit einem Verhältnis (II) : (III) von 1: 3,5.

### Beispiel 5

### Herstellung von all-trans-4-[[(4-Propylcyclohexyl)methyl]oxy]-4'-penlyl-1,1'-bicyclohexan [Verbindung (I) mit R¹ = Propyl, R²= Pentyl, R³ = R⁴ = R⁵ = R⁶ = H, m=0, n = 1]

Es wird wie in Beispiel 1 vorgegangen, jedoch mit einem Verhältnis (II) / (III) von 1: 2,95 gegenüber 1: 4 in Beispiel 1, einem Verhältnis (III) : (IV) von 12:1 gegenüber 8:1 in Beispiel 1 sowie einer Maßstabsvergrößerung von 8.

### Beispiel 6

### Herstellung von all-trans-4-[[(4-Propylcyclohexyl)oxy]methyl]-4-propyl-cyclohexan [Verbindung (I) mit R¹ =R²= Propyl, R³ = R⁴ = R⁵ = R⁶ = H, m = n = Null]

Eine Lösung von 29,4g (100 mmol) *all-trans-* 4-Propyl-cyclohexan-4-(4-propylcyclohexyl)carbonsäureester [(II) mit R¹ = R²= Propyl, m = n = Null)] in 50 ml Tetrahydrofuran wird mit 33,9g (250 mmol) Trichlorsilan (III) sowie 4,4g (29,74 mmol) Di-tertbutylperoxid (IV) versetzt und für 4 h bei 20°C dem Licht einer Mitteldruck-UV-Lampe (Tauchlampe, Quarzrohr, 150W) ausgesetzt. Anschließend wird die Reaktionsmischung auf 300 ml Eiswasser gegossen, 70 ml 32%ige Natriumhydroxidlösung werden zugesetzt, und es wird mit 100 ml tert-Butylmethylether extrahiert. Die wäßrige Phase wird noch zweimal mit je 100 ml tert-Butylmethylether extrahiert.Die organischen Phasen werden vereinigt und die Lösemittel werden im Vakuum abdestilliert. Das Rohprodukt wird der Analytik per GC unterzogen.

### Vergleichsbeispiel V1

### Herstellung von all-trans-4-[[(4-Propylcyclohexyl)methyl]oxy]-4'-pentyl-1,1'-bicyclohexan [Verbindung (I) mit R¹ = Propyl, R²= Pentyl, R³ = R⁴ = R⁵ = R⁶ = H, m=0, n = 1] nach EP-A-1 061 113

Eine Lösung von 26,26 g (65 mmol) *all-trans-* 4-Propylcyclohexancarbonsäure-4-(4'-pentyl-1,1'- bicyclohexan]ester [(II) mit R¹ = Propyl, R²= Pentyl, R³ = R⁴ = R⁵ = R⁶ = H, m=0, n = 1] in 300 ml Tetrahydrofuran wird bei 15 - 25 °C tropfenweise mit 31,8 g (227,5 mmol) Bortrifluorid-THF-Komplex versetzt. Nach beendeter Zugabe wird bei gleicher Temperatur eine Lösung von 6,14g (162,5 mmol) Natriumborhydrid in 150 ml Diethylenglykoldimethylether zugetropft. Anschließend wird für weitere 4 h bei 20°C gerührt. Es wird auf 2 l eiskalte 5%ige Natriumhydroxidlösung gegossen, dreimal mit je 100 ml tert-Butylmethylether extrahiert, die organischen Phasen werden vereinigt, mit gesättigter Natriumchloridlösung gewaschen, mit Natriumsulfat getrocknet; die Lösemittel werden im Vakuum abdestilliert. Das Rohprodukt wird der Analytik per GC unterzogen.

### Vergleichsbeispiel V2

### Herstellung von all-trans-4-[[(4-Propylcyclolzexyl)methyl]oxy]-4'-pentyl-1,1'-bicyclohexan [Verbindung (I) mit R¹ = Propyl, R²= Pentyl, R³ = R⁴ = R⁵ = R⁶ = H, m=0, n = 1] nach US 4,361,494

Zur Suspension von 1,56g (65 mmol) Natriumhydrid in 30 ml Tetrahydrofuran wird im Verlauf von 30 min eine Lösung von 16,4g (65 mmol) *trans*-4-Hydroxy-4'-pentyl-1,1' bicyclohexan [(XI) mit n=1, R²=Pentyl, R⁵=R⁶=H] in 20 ml Tetrahydrofuran getropft. Nach Rühren über Nacht wird eine Lösung von 14,2 g (65 mmol) *trans -* (4-Propylcyclohexyl)methylbromid [(XX) mit m=0, R¹= Propyl, R³=R⁴=H] in 30 ml Tetrahydrofuran zugetropft und die Mischung wird anschließend für 2 h zum Rückfluß erhitzt. Das Lösemittel wird im Vakuum abdestilliert, der Rückstand wird auf Eiswasser gegeben und dreimal mit je 100 ml Diethylether extrahiert. Die vereinigten organischen Phasen werden mit gesättigter Natriumchloridlösung gewaschen und mit Natriumsulfat getrocknet; die Lösemittel werden im Vakuum abdestilliert. Das Rohprodukt wird der Analytik per GC unterzogen.

### Vergleichsbeispiel V3

### Herstellung von all-trans-4-[[(4-Propylcyclohexyl)oxy]methyl]-4-propyl-cyclohexan [Verbindung (I) mit R¹ =R²= Propyl, R³ = R⁴ = R⁵ = R⁶ = H, m = n = Null] nach EP-A-1 061 113

Eine Lösung von 29,4 g (100 mmol) *all-trans-* 4-Propylcyclohexan-4-(4-propylcyclohexyl)carbonsäureester [(II) mit R¹ = R²= Propyl, R³ = R⁴ = R⁵ = R⁶ = H, m = n = Null)] in 300 ml Tetrahydrofuran wird bei 15 - 25 °C tropfenweise mit 48,9g (350 mmol) Bortrifluorid-THF-Komplex versetzt. Nach beendeter Zugabe wird bei gleicher Temperatur eine Lösung von 9,45g (250 mmol) Natriumborhydrid in 230 ml Diethylenglykoldimethylether zugetropft. Anschließend wird für weitere 4 h bei 20°C gerührt. Es wird auf 3 l eiskalte 5%ige Natriumhydroxidlösung gegossen, dreimal mit je 150 ml tert-Butylmethylether extrahiert, die organischen Phasen werden vereinigt, mit gesättigter Natriumchloridlösung gewaschen, mit Natriumsulfat getrocknet, und die Lösemittel werden im Vakuum abdestilliert. Das Rohprodukt wird der Analytik per GC unterzogen.

### Vergleichsbeispiel V4

### Herstellung von all-trans-4-[[(4-Propylcyclohexyl)oxy]methyl]-4-propyl-cyclohexan (Verbindung (I) mit R¹ =R²= Propyl, R³ = R⁴ = R⁵ = R⁶ = H, m = n = Null) nach US 4,361,494

Zur Suspension von 2,54g (100mmol) Natriumhydrid in 50 ml Tetrahydrofuran wird im Verlauf von 30 min eine Lösung von 14,2g (100 mmol) *trans*-4-Propylcyclohexanol [(XI) mit n=Null, R²=Propyl, R⁵ = R⁶ = H] in 30 ml Tetrahydrofuran getropft. Nach Rühren über Nacht wird eine Lösung von 21,9 g (100 mmol) *trans* -1-Brommethyl-4-propyl-cyclohexan [(XX) mit m=0, R¹=Propyl, R³ = R⁴ = H] in 30 ml Tetrahydrofuran zugetropft, und die Mischung wird anschließend für 2 h zum Rückfluß erhitzt. Das Lösemittel wird im Vakuum abdestilliert, der Rückstand auf Eiswasser gegeben und dreimal mit je 100 ml Diethylether extrahiert. Die vereinigten organischen Phasen werden mit gesättigter Natriumchloridlösung gewaschen, mit Natriumsulfat getrocknet, und die Lösemittel werden im Vakuum abdestilliert. Das Rohprodukt wird der Analytik per GC unterzogen.

Die GC-Analytik und die Ausbeuten sind in Tabelle 1 zusammengefasst.

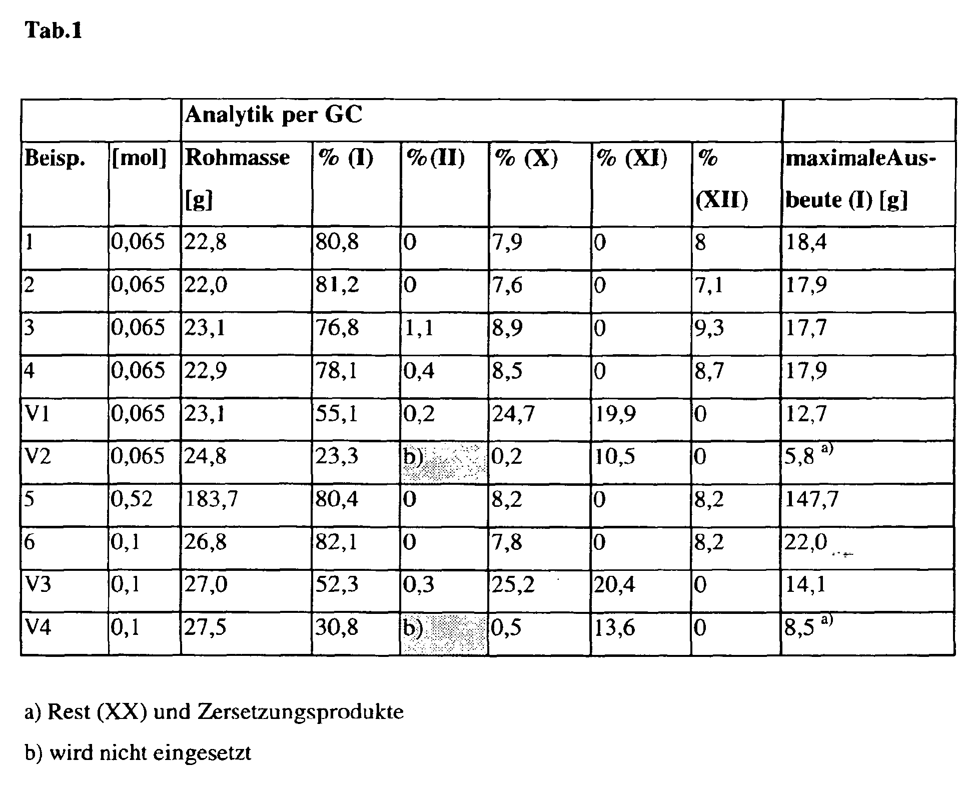

Tab.1 belegt mit den Vergleichswerten {Beispiele 1-4 vs. V1-V2; Beispiel 6 vs. V3-4 bei Anwendung auf eine andere Verbindung}, dass mit dem erfindungsgemäßen Verfahren höhere Gehalte der Zielverbindung (I) erhalten werden können bei gleichzeitig vergleichbarer bzw. höherer Rohmasse, so dass die Ausbeute an (I) [d.h. Rohmasse x Gehalt] deutlich höher ist als bei den Vergleichsverfahren. Beispiel 5 zeigt für dasselbe Zielmolekül wie in Beispielen 1 - 4 die Invarianz des Verfahrens hinsichtlich einer Maßstabsvergrößerung.

Die Raum-Zeit-Ausbeuten sind in Tabelle 2 zusammengefasst.

**Tab.2**

| **Versuch** | **Volumen Lösemittel [l]** | **Reaktionsszeit [h]** | **max.Ausb. [g]** | **Raum-Zeit-ausbeute [gl**^{**-1**}**h**^{**-1**}**]** |
|---|---|---|---|---|
| 1 | 0,13 | 3 | 18,4 | 47,2 |
| 2 | 0,13 | 3 | 17,9 | 45,9 |
| 3 | 0,13 | 9 | 17,7 | 15,1 |
| 4 | 0,13 | 6 | 17,9 | 22,9 |
| V1 | 0,45 | 5 | 12,7 | 5,6 |
| V2 | 0,11 | 16 | 5,8 | 3,3 |
| 5 | 1,04 | 3 | 147,7 | 47,3 |
| 6 | 0,05 | 4 | 22,0 | 110 |
| V3 | 0,53 | 5 | 14,1 | 1,7 |
| V4 | 0,11 | 16 | 8,5 | 4,8 |

Tab.2 belegt mit den Vergleichswerten {Beispiele 1-5 vs. V1-V2; Beispiel 6 vs. V3-4 bei Anwendung auf eine andere Verbindung;}, dass mit dem erfindungsgemäßen Verfahren höhere Raum-Zeit-Ausbeuten erzielt werden können, was einer deutlichen Verbesserung der Ökonomie des Verfahrens entspricht.

## Patentansprüche

1. Verfahren zur Herstellung von Ether-Verbindungen der Formel (I) 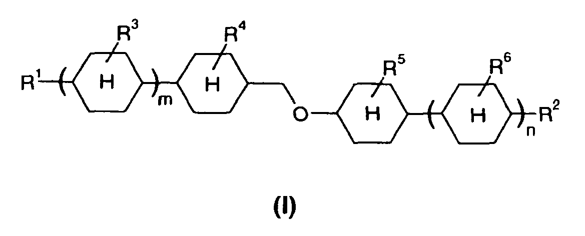 durch Reduktion von Carbonsäure-Estern der Formel (II), 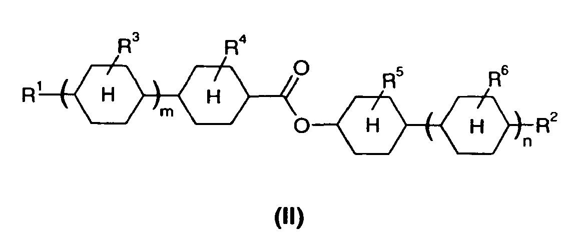 **dadurch gekennzeichnet, dass** 1 Mol-Äquivalent einer Verbindung der Formel (II) mit 2 bis 6 Mol-Äquivalenten eines Silans der Formel (III),
**(III) :** R¹¹R¹²R¹³SiH
in einer Radikalkettenreaktion, gegebenenfalls in einem Lösemittel, umgesetzt wird, wobei in den Formeln die Reste bedeuten:
**R**^{**1**}**, R**^{**2**} **:** jeweils unabhängig voneinander Wasserstoff oder einen unsubstituierten oder einfach durch CN oder CF₃ oder mindestens einfach durch Halogen substituierten Alkyl-Rest mit 1 bis 12 C-Atomen oder Alkenyl-Rest mit 2 bis 12 C-Atomen, wobei in diesen Resten auch eine oder mehrere -CH₂-Gruppen jeweils unabhängig voneinander durch -O-, -S-, Cyclopropan-1,2-diyl, Cyclobutan-1,3-diyl so ersetzt sein können, dass O-Atome nicht direkt miteinander verknüpft sind, mit der Maßgabe, dass von R¹, R² nur eines Wasserstoff sein darf
**R**^{**3**}**, R**^{**4**}**, R**^{**5**}**, R**^{**6**} jeweils unabhängig voneinander Wasserstoff, Halogen, Methyl oder Cyano
**R**^{**11**}**, R**^{**12**}**, R**^{**13**} jeweils unabhängig voneinander Wasserstoff, Chlor, Alkyl mit 1 bis 8 C-Atomen, Alkyloxy mit 1 bis 8 C-Atomen, Cyclohexyl, Methylcyclohexyl, Cyclopentyl, Phenyl, oder R¹¹ und R¹² zusammen die Gruppierung -(CH₂)ₚ- ,
mit der Maßgabe, dass mindestens eines von R¹¹, R¹², R¹³ Chlor bedeutet,
**m, n** jeweils unabhängig voneinander Null oder 1,
**p** 4 oder 5.

2. Verfahren nach Anspruch 1, **dadurch gekennzeichnet, dass** das Verhältnis der Mol-Äquivalente von (II) : (III) im Bereich von 1:2 bis 1: 5 liegt.

3. Verfahren nach Anspruch 1 oder 2, **dadurch gekennzeichnet, dass** die Radikalkettenreaktion durch freie Radikale ausgelöst wird, die bei der Umsetzung durch Zerfall von Radikalstartern, durch aktinische Strahlung oder eine Kombination davon, gebildet werden.

4. Verfahren nach Anspruch 3, **dadurch gekennzeichnet, dass** Radikalstarter eingesetzt werden, die thermisch und/oder photochemisch unter Bildung freier Radikale zerfallen.

5. Verfahren nach Anspruch 4, **dadurch gekennzeichnet, dass** Radikalstarter der Formeln (IV) bzw. (V) eingesetzt werden
**(IV)**: R²¹OOR²²
**(V)**: R³¹-N=N-R³²
mit der Bedeutung
**R**^{**21**} **, R**^{**22**} Wasserstoff oder ein (gegebenenfalls durch Alkylperoxy- oder Hydroperoxy-Gruppen substituierter) Alkyl- oder Alkandiyl-Rest mit 1 bis 8 C-Atomen oder ein (gegebenenfalls durch Alkyl- und/oder Alkylperoxy- und/oder Hydroxy- und/oder Hydroperoxy-Gruppen substituierter) Cyclohexanring, mit der Maßgabe, dass nur eines von R²¹ , R²² Wasserstoff bedeuten kann
**R**^{**31**} **, R**^{**32**} jeweils unabhängig voneinander R⁴¹R⁴²C(X), worin R⁴¹, R⁴² unabhängig voneinander eine Alkyl- oder Alkyloxygruppe mit 1 bis 8 C-Atomen und X -CN oder -C(=O)OR⁴³ bedeutet, mit R⁴³ = Alkylgruppe mit 1 bis 8 C-Atomen.

6. Verfahren nach einem der Ansprüche 1 bis 5, **dadurch gekennzeichnet, dass** im Silan der Formel (III) R¹¹ und R¹² Chlor bedeuten.

7. Verfahren nach einem der Ansprüche 1 bis 6, **dadurch gekennzeichnet, dass** die Umsetzung in einem inerten Lösungsmittel, ausgewählt aus Ethern, Kohlenwasserstoffen, Chlorkohlenwasserstoffen oder Gemischen davon, durchgeführt wird.

8. Verfahren nach einem der Ansprüche 1 bis 7, **dadurch gekennzeichnet, dass** in den Verbindungen der Formeln (I) bzw. (II) R¹ und R² C₂₋₇-Alkyl und R³, R⁴, R⁵, R⁶ unabhängig voneinander Wasserstoff oder Halogen bedeuten.

9. Verfahren nach einem der Ansprüche 1 bis 8, **dadurch gekennzeichnet, dass** in den Verbindungen der Formeln (I) bzw. (II) m + n Null oder 1 bedeutet.

10. Verfahren zur Herstellung von Ether-Verbindungen der Formel (XXI) 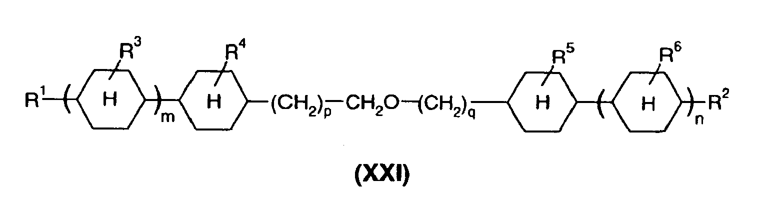 durch Reduktion von Carbonsäure-Estern der Formel (XXII), 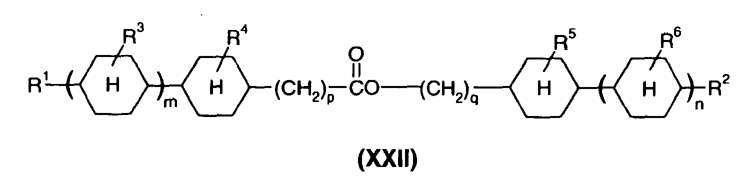 **dadurch gekennzeichnet, dass** 1 Mol-Äquivalent einer Verbindung der Formel (XXII) mit 2 bis 6 Mol-Äquivalenten eines Silans der Formel (III)
**(III)** R¹¹R¹²R¹³SiH
in einer Radikalkettenreaktion, gegebenenfalls in einem Lösemittel, umgesetzt wird; wobei in den Formeln die Reste bedeuten:
**R**^{**1**}**, R**^{**2**} **:** jeweils unabhängig voneinander Wasserstoff oder einen unsubstituierten oder einfach durch CN oder CF₃ oder mindestens einfach durch Halogen substituierten Alkyl-Rest mit 1 bis 12 C-Atomen oder Alkenyl-Rest mit 2 bis 12 C-Atomen, wobei in diesen Resten auch eine oder mehrere -CH₂-Gruppen jeweils unabhängig voneinander durch -O-, -S-, Cyclopropan-1,2-diyl, Cyclobutan-1,3-diyl so ersetzt sein können, dass O-Atome nicht direkt miteinander verknüpft sind, mit der Maßgabe, dass von R¹, R² nur eines Wasserstoff sein darf
**R**^{**3**}**, R**^{**4**} **, R**^{**5**}**, R**^{**6**} jeweils unabhängig voneinander Wasserstoff, Halogen, Methyl oder Cyano
**R**^{**11**}**, R**^{**12**}**, R**^{**13**} jeweils unabhängig voneinander Wasserstoff, Chlor, Alkyl mit 1 bis 8 C-Atomen, Alkyloxy mit 1 bis 8 C-Atomen, Cyclohexyl, Methylcyclohexyl, Cyclopentyl, Phenyl,
mit der Maßgabe, dass mindestens eines von R¹¹, R¹², R¹³ Chlor bedeutet,
**m, n** jeweils unabhängig voneinander Null oder 1,
**p und q** jeweils unabhängig voneinander 0, 1, 2 oder 3, die Summe p+q 1, 2 oder 3.

## Claims

1. A process for preparing ether compounds of the formula (I) by reducing carboxylic acid compounds of the formula (II), 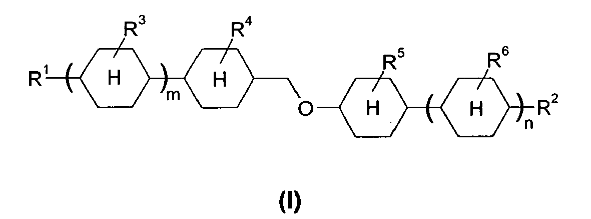 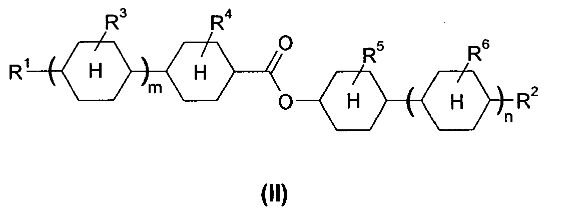 which comprises reacting 1 molar equivalent of a compound of the formula (II) with from 2 to 6 molar equivalents of a silane of the formula (III)
**(III) :** R¹¹R¹²R¹³SiH
in a free-radical chain reaction, optionally in a solvent, where the radicals in the formulae are:
**R**^{**1**}**, R**^{**2**} **:** each independently hydrogen or an unsubstituted, a mono-CN- or -CF₃- substituted or an at least mono-halogen-substituted alkyl radical having from 1 to 12 carbon atoms or alkenyl radical having from 2 to 12 carbon atoms, and one or more -CH₂- groups in these radicals may also each independently be replaced by -O-, -S-, cyclopropane-1,2-diyl, cyclobutane-1,3-diyl, in such a way that oxygen atoms are not bonded directly to one another, with the proviso that only one of R¹, R² can be hydrogen,
**R**^{**3**}**, R**^{**4**}**, R**^{**5**}**, R**^{**6**} are each independently hydrogen, halogen, methyl or cyano,
**R**^{**11**}**, R**^{**12**}**, R**^{**13**} are each independently hydrogen, chlorine, alkyl having from 1 to 8 carbon atoms, alkyloxy having from 1 to 8 carbon atoms, cyclohexyl, methylcyclohexyl, cyclopentyl, phenyl, or R¹¹ and R¹² together are the -(CH₂)ₚ- molarity, with the proviso that at least one of R¹¹, R¹², R¹³ is chlorine,
**m, n** are each independently zero or 1,
**p** is 4 or 5.

2. The process as claimed in claim 1, wherein the ratio of molar equivalents of (II) : (III) is in the range from 1:2 to 1: 5.

3. The process as claimed in claim 1 or 2, wherein the free-radical chain reaction is initiated by free radicals which are formed in the reaction by decomposition of free-radical initiators, by actinic radiation or a combination thereof.

4. The process as claimed in claim 3, wherein free-radical initiators are used which decompose thermally and/or photochemically to form free radicals.

5. The process as claimed in claim 4, wherein free-radical initiators of the formula (IV) or (V) are used
**(IV)**: R²¹OOR²²
**(V)**: R³¹-N=N-R³²
where
**R**^{**21**}**, R**^{**22**} are each hydrogen or an (optionally alkylperoxy- or hydroperoxy-substituted) alkyl or alkanediyl radical having from 1 to 8 carbon atoms or an (optionally alkyl- and/or alkylperoxy- and/or hydroxy- and/or hydroperoxy-substituted) cyclohexane ring, with the proviso that only one of R²¹, R ²² may be hydrogen,
**R**^{**31**} **, R**^{**32**} are each independently R⁴¹R⁴²C(X) where R⁴¹, R⁴² are each independently an alkyl or alkyloxy group having from 1 to 8 carbon atoms and X is -CN or -C(=O)OR⁴³ where R⁴³ = alkyl group having from 1 to 8 carbon atoms.

6. The process as claimed in one of claims 1 to 5, wherein R¹¹ and R¹² in the silane of the formula (III) are each chlorine.

7. The process as claimed in one of claims 1 to 6, wherein the reaction is carried out in an inert solvent selected from ethers, hydrocarbons, chlorohydrocarbons and mixtures thereof.

8. The process as claimed in one of claims 1 to 7, wherein R¹ and R² in the compounds of the formulae (I) and (II) are each C₂₋₇-alkyl, and R³, R⁴, R⁵, R⁶ are each independently hydrogen or halogen.

9. The process as claimed in one of claims 1 to 8, wherein m + n in the compounds of the formulae (I) and (II) is zero or 1.

10. A process for preparing ether compounds of the formula (XXI) by reducing carboxylic acid compounds of the formula (XXII), 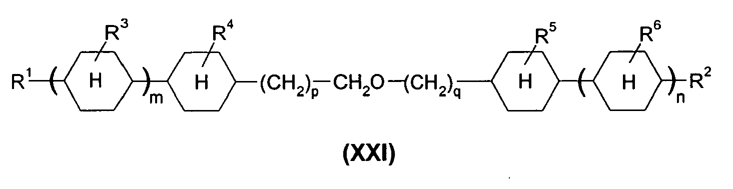 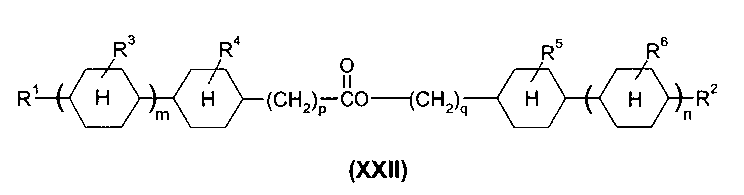 which comprises reacting 1 molar equivalent of a compound of the formula (XXII) with from 2 to 6 molar equivalents of a silane of the formula (III)
**(III) :** R¹¹R¹²R¹³SiH
in a free-radical chain reaction, optionally in a solvent, where the radicals in the formulae are:
**R**^{**1**}**, R**^{**2**} **:** each independently hydrogen or an unsubstituted, a mono-CN- or -CF₃- substituted or an at least mono-halogen-substituted alkyl radical having from 1 to 12 carbon atoms or alkenyl radical having from 2 to 12 carbon atoms, and one or more -CH₂- groups in these radicals may also each independently be replaced by -O-, -S-, cyclopropane-1,2-diyl, cyclobutane-1,3-diyl, in such a way that oxygen atoms are not bonded directly to one another, with the proviso that only one of R¹, R² can be hydrogen,
**R**^{**3**}**, R**^{**4**}**, R**^{**5**}**, R**^{**6**} are each independently hydrogen, halogen, methyl or cyano,
**R**^{**11**}**, R**^{**12**}**, R**^{**13**} are each independently hydrogen, chlorine, alkyl having from 1 to 8 carbon atoms, alkyloxy having from 1 to 8 carbon atoms, cyclohexyl, methylcyclohexyl, cyclopentyl, phenyl, with the proviso that at least one of R¹¹, R¹²,
R¹³ is chlorine,
**m, n** are each independently zero or 1,
**p and q** each independently 0, 1, 2 or 3, the sum p+q being 1, 2 or 3.

## Revendications

1. Procédé pour la préparation de composés éthers de formule (I) 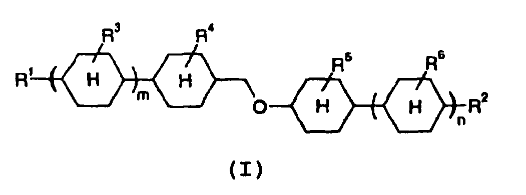 par réduction d'esters d'acide carboxylique de formule (II), 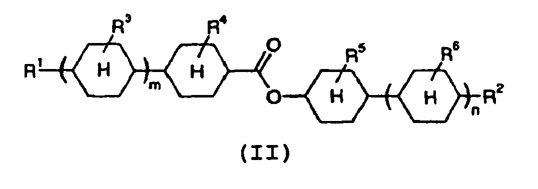 **caractérisé en ce que** l'on met à réagir 1 équivalent en moles d'un composé de formule (II) avec 2 à 6 équivalents en moles d'un silane de formule (III),
(**III**) : **R**^{**11**}**R**^{**12**}**R**^{**13**}**SiH**
dans une réaction radicalaire en chaîne, éventuellement dans un solvant, formules dans lesquelles les radicaux :
R¹, R² représentent indépendamment l'une de l'autre un hydrogène ou un radical alkyle avec 1 à 12 atomes de carbone ou un radical alcényle avec 2 à 12 atomes de carbone non substitué ou substitué une fois par CN ou CF₃ ou au moins une fois par un halogène, dans lesquels dans ces radicaux également un ou plusieurs groupes -CH₂- peuvent être remplacés respectivement indépendamment les uns des autres par -O-, -S-, un cyclopropane-1,2-diyle, cyclobutane-1,3-diyle, de sorte que les atomes O ne soient pas directement reliés entre eux, sous réserve que seulement un de R¹, R² doive être un hydrogène
R³, R⁴, R⁵, R⁶ représentent respectivement indépendamment les uns des autres un hydrogène, halogène, méthyle ou cyano
R¹¹, R¹², R¹³ représentent respectivement indépendamment les uns des autres un hydrogène, chlore, alkyle avec 1 à 8 atomes de carbone, alkyloxy avec 1 à 8 atomes de carbone, cyclohexyle, méthylcyclohexyle, cyclopentyle, phényle, ou R¹¹ et R¹² forment ensemble le groupement -(CH₂)ₚ-, sous réserve qu'au moins l'un de R¹¹, R¹², R¹³ représente un chlore,
m, n représentent indépendamment l'un de l'autre zéro ou 1,
p 4 ou 5.

2. Procédé selon la revendication 1, **caractérisé en ce que** le rapport des équivalents en moles de (II) : (III) se situe dans la gamme de 1 : 2 à 1 : 5.

3. Procédé selon la revendication 1 ou 2, **caractérisé en ce que** la réaction radicalaire en chaîne est démarrée au moyen de radicaux libres, qui sont formés au cours de la réaction par la décomposition d'amorceurs radicalaires, par un rayonnement actinique ou une combinaison de ces effets.

4. Procédé selon la revendication 3, **caractérisé en ce qu'**on utilise des amorceurs radicalaires qui se décomposent thermiquement et/ou photochimiquement en formant des radicaux libres.

5. Procédé selon la revendication 4, **caractérisé en ce qu'**on utilise des amorceurs radicalaires de formule (IV) ou (V)
(IV) R²¹OOR²²
(V) R³¹-N=N-R³²
dans lesquelles
R²¹, R²² représentent un hydrogène ou un radical alkyle ou alcanediyle (éventuellement substitué par des groupes alkylperoxy ou hydroperoxy) avec 1 à 8 atomes de carbone ou un cycle cyclohexane (éventuellement substitué par des groupes alkyle et/ou alkylperoxy et/ou hydroxy et/ou hydroperoxy), sous réserve que seul l'un de R²¹, R²² puisse représenter un hydrogène
R³¹, R³² représentent indépendamment l'un de l'autre R⁴¹R⁴²C(X), dans lequel R⁴¹, R⁴² indépendamment l'un de l'autre représentent un groupe alkyle ou alkyloxy avec 1 à 8 atomes de carbone et X-CN ou -C(=O)OR⁴³, avec R⁴³ = un groupe alkyle avec 1 à 8 atomes de carbone.

6. Procédé selon une des revendications 1 à 5, **caractérisé en ce que** dans le silane de formule (III) R¹¹ et R¹² représentent le chlore.

7. Procédé selon l'une des revendications 1 à 6, **caractérisé en ce que** l'on effectue la réaction dans un solvant inerte, choisi parmi les éthers, les hydrocarbures, les chlorohydrocarbures ou leurs mélanges.

8. Procédé selon l'une des revendications 1 à 7, **caractérisé en ce que** dans les composés de formules (I) et (II) R¹ et R² représentent un alkyle en C₂ à C₇ et R³, R⁴, R⁵, R⁶ représentent indépendamment les uns des autres un hydrogène ou halogène.

9. Procédé selon l'une des revendications 1 à 8, **caractérisé en ce que** dans les composés de formules (I) et (II) m + n représente zéro ou 1.

10. Procédé de préparation de composés éther de formule (XXI) 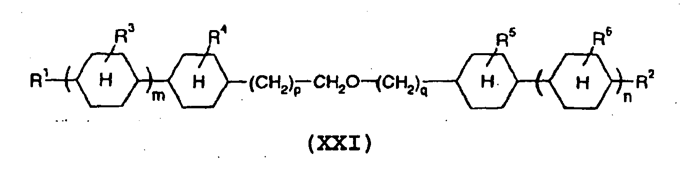 par réduction d'esters d'acide carboxylique de formule (XXII) 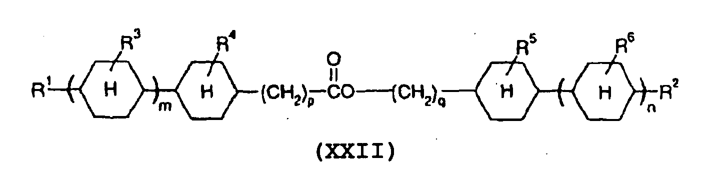 **caractérisé en ce que** l'on met à réagir 1 équivalent en moles d'un composé de formule (XXII) avec 2 à 6 équivalents en moles d'un silane de formule (III)
(III) R¹¹R¹²R¹³SiH
dans une réaction en chaîne radicalaire, éventuellement dans un solvant ;
formules dans lesquelles
R¹, R² représentent indépendamment l'une de l'autre un hydrogène ou un radical alkyle avec 1 à 12 atomes de carbone ou un radical alcényle avec 2 à 12 atomes de carbone non substitué ou substitué une fois par CN ou CF₃ ou au moins une fois par un halogène, dans lesquels dans ces radicaux également un ou plusieurs groupes -CH₂- peuvent être remplacés respectivement indépendamment les uns des autres par -O-, -S-, un cyclopropane-1,2-diyle, cyclobutane-1,3-diyle, de sorte que les atomes O ne soient pas directement reliés entre eux, sous réserve que seulement un de R¹, R² doive être un hydrogène
R³, R⁴, R⁵, R⁶ représentent respectivement indépendamment les uns des autres un hydrogène, halogène, méthyle ou cyano
R¹¹, R¹², R¹³ représentent respectivement indépendamment les uns des autres un hydrogène, chlore, alkyle avec 1 à 8 atomes de carbone, alkyloxy avec 1 à 8 atomes de carbone, cyclohexyle, méthylcyclohexyle, cyclopentyle, phényle, sous réserve qu'au moins l'un de R¹¹, R¹², R¹³ représente un chlore,
m, n représentent indépendamment l'un de l'autre zéro ou 1,
p et représentent indépendamment l'un de l'autre 0, 1, 2 ou 3, la somme p + q 1, 2 ou 3.
